# EUROPEAN PATENT APPLICATION

(11) **EP 0 586 112 A2**
(43) Date of publication of application: **09.03.1994**
(21) Application number: 93306300.0
(22) Date of filing: 10.08.1993
(51) Int. Cl.: C12N 15/11, C12Q 1/68

(54) **Control of PCR mediated detection of micro-organisms**

(30) Priority: 14.08.1992 GB 9217368; 22.02.1993 GB 9303498; 05.05.1993 GB 9309198; 05.05.1993 GB 9309195
(71) Applicant: PHARMA GEN, S.A., E-28760 Tres Cantos (ES)
(72) Inventor: Tercero, Juan Carlos, c/o Pharma Gen S.A, 28760 Tres Cantos (Madrid) (ES); Garcia, Lucia, c/o Dept. de Bioquimica, E-28040 Madrid (ES); Ramos, Jose Antonio, c/o Dept. de Bioquimica, E-28040 Madrid (ES); Alemany, Jorge, c/o Pharma Gen S.A., E-28760 Tres Cantos (Madrid) (ES)
(74) Representative: Harrison, Susan Joan

(57) **Abstract**

A vector is provided which acts as a positive control in the PCR mediated detection of nucleic acids in a sample, and in particular of micro-organisms in a clinical sample, thereby eliminating the occurrence of false negative results.

## Description

### Background to the Invention

The present invention relates to a vector for use as a positive control in the detection of a nucleic acid sequence in a sample via amplification using the polymerase chain reaction, as well as to a process for such detection of a nucleic acid sequence.

Prior to the development of the polymerase chain reaction (PCR) amplification of nucleic acid, there were many methods for the identification of micro-organisms, including:
1. by direct visualisation using microscopy:- although this method is rapid, it requires a high concentration of the micro-organism, for example of more than 10⁴ bacteria per ml of sample, in order to obtain a positive result;
2. by culture:- certain micro-organisms can be identified by culturing of a sample, although this technique can be very slow for certain micro-organisms, requiring a minimum of three weeks, and can also be dangerous, depending on the micro-organism in question;
3. by hybridization with RNA or DNA labelled probes:- this again requires a high concentration of the bacteria in question and is therefore rarely applicable to clinical samples;
4. serologic techniques:- these can be less specific and therefore the least reliable; and
5. immunological techniques, for example using immunofluorescence.

The PCR is a well used technique for the amplification of target nucleic acid sequences. The technique, which is described in US Patent Specifications No. 4,683,195 and 4,683,202, the disclosures of which are incorporated herein by reference, involves the hybridization of an added primer sequence to a target nucleic acid sequence to be amplified, followed by formation of an extension product of the primer having a sequence complementary to that of the target. Because of the presence of excess primer and the constant separation of double stranded nucleic acid, the amplification is exponential. Although the PCR is normally carried out on DNA samples, by the inclusion of a step for the transcription of RNA into DNA, RNA sequences may also be amplified using the PCR technique.

In this manner nucleic acid sequences which can otherwise only be isolated in extremely small amounts, can be amplified, thereby providing much larger quantities for later manipulation.

The PCR technique has found widespread application in diagnosis and, in particular, in the diagnosis of genetic diseases, cancer and infectious diseases, as well as in the determination of disease susceptibility. However, the diagnosis of diseases such as infections caused by *Mycobacterium tuberculosis* and *Mycobacterium bovis*, the identification of the pathogens causing periodontitis and the identification of the causes of many viral infections is still an important clinical problem which needs to be addressed.

There are now a large number of PCR methods which allow the identification of both double and single stranded RNA viruses, double stranded RNA virus transcripts and the detection and quantification of cellular live infective micro-organism messenger RNA's (mRNA). Because PCR identification relies on amplification, only a minute amount of the sample is necessary. This makes this technique particularly suitable for diagnosing diseases directly from clinical samples such as blood, sputum, urine, spinal chord fluid and any other tissues.

The diagnosis of infectious agents using the PCR technique is described by, for example, Eisenstein in N. Eng. J. Med. 322 (1990) 178. Furthermore, the identification of strains of *Mycobacterium tuberculosis* and *Mycobacerium bovis* by the PCR amplification of different genes has been described in the literature. Thus, the amplification of the ELgro gene is described by Hance et al in Mol. Microbiol. 3 (1989) 843; the amplification of the b antigen by Sjöbring et al, in J. Clin. Microbiol. 28 (1990) 2200; the amplification of the oligonucleotide IS6110 by Eisenach et al, in J. Infectious Dis., 161 (1990) 977; the amplification of IS986 by Hermans et al, in J. Clin. Microbiol. 28 (1990) 2051; the amplification of the 65KDa antigen by Pao et al in J. Clin. Microbiol., 28 (1990) 1877; the amplification of rRNA by Boddinghaus et al in J. Clin. Microbiol, 28 (1990) 1751 and the amplification of PH7311 by Hermans et al in J. Clin. Microbiol. 28 (1990) 1204.

However, even though the PCR has been used successfully in all of these applications, there is still- one major drawback to its routine use in diagnosis and, in particular, diagnosis of infectious diseases. This drawback is the probability of obtaining either false positive or false negative results. False positive results are normally due to the cross-contamination of the samples with the amplified product. Such cross-contamination can be circumvented with relative ease, for example, by the use of good laboratory practices, such as the physical separation of the DNA extraction steps from the manipulation of the amplified products, or by the use of disposable pipettes and other disposable materials.

On the other hand, false negative results, that is when the test results indicate wrongly that there is no target nucleic acid in the sample, are obtained when the PCR reaction itself does not work properly. Such results may be obtained when, for example, the reactions are spoiled, the manipulation is wrong, there is RNase in the reverse-transcriptase PCR (RT-PCR) sample or, most frequently, when there are inhibitors of the PCR in the reaction sample. Thus, for example, it is known that sputum, which is the clinical sample most commonly tested for micro-organisms such as the Mycobacteria, contains inhibitors of the PCR.

Clearly the possibility of obtaining false negative results in the PCR mediated diagnosis of infectious micro-organisms is a serious deterrent to the use of this technique, when the risk of obtaining such results leaves the tester unsure as to whether the results are negative because there is no target nucleic acid in the sample, or because the amplification process has somehow been inhibited.

Although the occurrence of false negative results in the PCR amplification of nucleic acid samples is frequent, and has often been described in the literature, there is, as yet, no reliable method for the prevention of such results.

It is an object of the present invention to prevent the occurrence of false negative results in the PCR amplification of nucleic acid samples.

It is a further object of the present invention to enable the identification of infectious micro-organisms directly from clinical samples.

It is yet a further object of the present invention to. provide a process for the identification of a micro-organism using the PCR amplification technique without the risk of obtaining false negative results.

These and other objects of the invention will become clear from the following description.

### Brief Description of the Invention

In accordance with the above, the present invention provides a vector for use as a positive control in the detection of a particular nucleic acid sequence in a sample by the polymerase chain reaction amplification method, wherein the vector includes at least one nucleotide sequence comprising a sequence substantially identical to the sequence of a primer used in the polymerase chain reaction amplification method.

The present invention also provides the use of a vector including at least one nucleotide sequence comprising a sequence substantially identical to the sequence of a primer used in the polymerase chain reaction amplification method in the detection of a nucleic acid sequence using the polymerase chain reaction.

The present invention further provides a primer suitable for use in the PCR mediated detection of periodontitis pathogens.

The present invention furthermore provides a method for the detection of a particular nucleic acid in a sample, which method involves performance of the polymerase chain reaction on a sample suspected of containing said particular nucleic acid sequence in the presence of a positive control vector.

### Brief Description of the Drawings

Figure 1 shows, in diagrammatical form, the structure of plasmid pPG10.

Figure 2 shows a photograph of an ethidium bromide stained agarose gel of the amplification products formed during the detection of *Mycobacterium tuberculosis* and/or *Mycobacterium bovis* in the presence of the positive control plasmid pPG10. M is the molecular weight marker and columns 1 to 13 are the clinical samples analyzed.

Figure 3 shows,in diagrammatical form, the structure of plasmid pPGU3.

Figure 4 shows a photograph of an ethidium bromide stained agarose gel of the amplification products formed during the detection of bacteria causing periodontal disease in the presence of the positive control plasmid pPGU3. M is the molecular weight marker, columns 1 to 11 are the clinical samples analyzed and the column marked - is the negative control.

Figure 5 shows, in diagrammatical form, the structure of plasmid pPG17.

Figure 6 shows, in diagrammatical form, the structure of plasmid pPG22.

Figure 7 shows, in diagrammatical form, the structure of plasmid pPG26.

### Detailed Description of the Invention

The present invention provides, in a first aspect, a vector for use as a positive control in the PCR amplification of a nucleic acid sample. By acting as a positive control of the PCR amplification the particular nucloetide sequence in the vector is amplified at the same time as the target nucleic acid and using the same PCR conditions as used for amplification of that target nucleic acid. However, amplification of the sequence in the vector results in the production of an amplification product of a different size from that of the target nucleic acid. In this manner, the vector of the present invention substantially totally eliminates the occurrence of false negative results in the PCR mediated detection of a nucleic acid in a sample.

The vector of the present invention allows the control of the PCR mediated detection of a nucleic acid in a sample. The nucleic acid sample to be detected is, generally, derived from a micro-organism or infected cell. Typical nucleic acids which may be detected using the PCR amplification method and which may be subjected to positive control using the vector of the present invention include both DNA and RNA and may be, for example, derived from the genome of an infectious micro-organism, derived from the total nucleic acid of a cancerous cell, derived from the genome of a single stranded or double stranded virus, for example an RNA or DNA virus, or it may be a double stranded RNA virus transcript. Alternatively, or in addition, the nucleic acid may be the messenger RNA of a live infective micro-organism or eukaryotic cell.

The vector of the present invention may be any vector which is typically used in gene manipulation and which can be readily manipulated to include the particular nucleotide sequences which render it a positive control vector. Typical basic vectors, that is the vector before manipulation to include the particular nucleotide sequences which render it a positive control vector, include plasmids, viruses and bacteriophages, any or all of which may be replicons in their own right, or which may be artificially constructed using standard genetic engineering techniques.

The choice of the basic vector is not essential to the present invention. However, this choice may depend on, for example, the type of nucleic acid to be detected and/or the original source of that nucleic acid. Thus, for example, for the detection of a DNA target sequences in the PCR amplification method, it may be preferred to use a plasmid as vector, whereas for the detection of RNA target sequences, it may be more appropriate to use a recombinant RNA virus. Further factors which may influence the choice of vector include the availability of the specific vector, the size of the vector and the ease of manipulation of the vector.

In the present invention we particularly prefer to use a plasmid or an RNA virus as vector. Suitable plasmids which can be manipulated in order to transform them into positive control plasmids include pBLUESCRIPT SKII+™ (available from Stratagene), pBR322 and pUC19. Of these, we particularly prefer pBLUESCRIPT SKII+.

Suitable RNA viruses which may be manipulated in order to transform them into the positive control vector of the present invention include any double or single stranded RNA virus, depending on whether the aim is to detect double or single stranded RNA. Typically, therefore, a double stranded RNA viral molecule may be used as a positive control in the identification of double stranded viruses, whereas a single stranded RNA viral transcript may be used to detect single stranded RNA viruses or messenger RNAs. We particularly prefer to use defective yeast double stranded RNA viruses that are not able to infect yeast or any other cells, and which are not able to replicate into yeast without the aid of a helper phage. Such viruses include the X virus which is a double stranded RNA yeast virus, the L-A, L-BC or M1 viruses, of which we particularly prefer the X virus.

The vector of the present invention includes at least one nucleotide sequence which is substantially identical to the sequence of a primer used in the PCR amplification of a particular nucleic acid. The number of particular such nucleotide sequences contained in the vector is not essential to the present invention. Typically, the vector should contain only sufficient nucleotide sequences identical to a primer used in the PCR amplification to enable satisfactory elimination of the occurrence of false negative results. We have found that this aim is achieved when the vector contains from one to ten such sequences. However, we prefer that the vector should contain either one or two such nucleotide sequences.

As indicated above, the vector of the present invention contains at least one nucleotide sequence which is substantially identical to the sequence of a primer used in the PCR amplification of a nucleic acid target. It may be that more than two primers, each primer having a different sequence, are used in the PCR amplification of a nucleic acid. In such situations, it is not necessary for the sequence of each primer to be inserted also into the positive control vector.

When a multiple polymerase chain reaction is performed, for example to detect the presence of more than one micro-organism or nucleotide sequence, several pairs of primers specific for each microorganism to be detected may be used. In this particular case, a single positive control vector is enough to monitor the PCR reaction. For instance, in the detection of, for example, multiple periodontal pathogens, four primers may be used, in which case one primer is typically common to all of the pathogens, and the other three are specific for each micro-organism. The positive control vector for use with such a multiple PCR reaction may then include the sequence of the common primer alone, this primer being inserted into both the sense and anti-sense strands of the vector nucleic acid.

However, it is preferred that the vector of the present invention is so constructed as to be host specific, that is so as to enable the detection of a nucleic acid sequence derived from a particular micro-organism.

The nucleotide sequences contained in the vector of the present invention and which render that vector a positive control vector, are substantially identical to a primer used in the PCR amplification of a nucleic acid sequence. Although minor variations between the sequences inserted into the vector and existing in the PCR primer may be present without substantially affecting the ability of the vector to act as a positive control, it is preferred that such sequences are identical. Variations which do not have any substantial effect on the activity of the vector include a difference in length between the sequence of the PCR primer and the corresponding nucleotide sequence inserted into the vector, for example the sequence of the primer may be shorter than the sequence inserted into the vector or, alternatively, the sequence of the PCR primer may be longer than that inserted into the vector. However, we generally prefer that the sequences of the PCR primer and the corresponding sequence inserted into the vector are of the same length.

Because the nucleotide sequence contained in the vector to render it a positive control and the sequence of a PCR primer are substantially identical, any limitations on the PCR primer will likewise apply to the sequence contained in the vector. The construction or obtention of PCR primer sequences is well within the skill of the man in the art and is well documented in the art, for example as detailed in the Oligo (TM) Primer Analysis Software of Medprobe.

As discussed above, the vector of the present invention may contain at least one nucleotide sequence which is identical to a primer used in the PCR amplification method. When more than one such sequence is inserted into the vector, the sequences are suitably inserted at a predetermined distance from one another. Although the exact distance between the nucleotide sequences in the vector is not essential to the present invention, this distance should be larger, in terms of the number of base pairs, than the length of the nucleotide sequences. A further consideration in the determination of this distance is the fact that smaller amplification products, or target sequences, are favoured by the PCR amplification method. Therefore, because the aim is to detect a specific nucleic acid sequence, it is clearly desirable that the positive control nucleotide sequence is larger, and therefore less attractive to the PCR amplification, than the target nucleic acid sequence. Preferably, the distance between the primers in the vector should be less than 1.5 Kb. We particularly prefer that this distance is equivalent to about 3 times the length of the longest primer nucleotide sequence in the vector.

When the vector of the present invention includes more than one nucleotide sequence substantially identical to the sequence of a PCR primer, for example when that vector contains two such sequences, each sequence being substantially identical to the same or a different primer, then it is preferred that the two nucleotide sequences are inserted into the vector on opposite strands of the vector. Thus, for example, when using a plasmid as a vector, it is preferred that one such nucleotide sequence is inserted in the sense strand and the other nucleotide sequence is inserted in the anti-sense strand, a particular distance from the first strand, as discussed above.

In a further aspect, the present invention provides a method for the preparation of a vector for use as a positive control in the PCR mediated detection of a nucleic acid sequence, which method comprises inserting into an appropriate vector at least one nucleotide sequence, which nucleotide sequence is substantially identical with a primer used in the PCR amplification of a nucleic acid.

The particular nucleotide sequences of choice may be inserted into the basic vector using any standard genetic manipulation technique, and the precise technique chosen is not essential to the present invention. We have generally found that site directed mutagenesis is preferable for accurate insertion of the desired sequence into the vector, followed by selection for the resulting modified vector.

Viral vectors suitable for positive controls in the PCR method can be constructed using techniques standard in the art. Generally, if using a yeast virus, a yeast strain is transformed with a DNA plasmid containing the sequence of the virus, together with the positive control sequence(s). Selected transformants thus allow the replication and encapsulation of the plasmid transcripts containing the viral and positive controls sequence(s).

As mentioned above, suitable vectors are well known and well documented in the prior art. Furthermore, PCR primer sequences are known for a variety of target nucleic acid sequences. In the alternative, such PCR primer sequences may be constructed using standard DNA synthesis techniques, using information obtainable from the nucleic acid or micro-organism to be detected. Typically, such techniques involve the determination of a variable region within the DNA of the micro-organism to be detected. Primers may then be constructed on the basis of the information contained in this variable region. Further exemplification of such techniques are provided in Step A of Example 2, hereinafter.

In yet a further aspect, the present invention provides a method for the detection of a particular nucleic acid in a sample, which method involves perfomance of the polymerase chain reaction on a sample suspected of containing said particular nucleic acid sequence, in the presence of a positive control vector.

The positive control vector for use in the method of this aspect of the present invention is any such vector as hereinbefore described.

Methods for the obtention of samples from a patient and the collection of nucleic acid from such samples are well known and documented in the prior art. The sample from which the target nucleic acid is collected is not a limitation on the present invention. On the contrary, we have found that the present invention allows direct testing on nucleic acid obtained from any typical clinical sample. Such samples include urine, blood, sputum and cerebrospinal fluid. Techniques for the collection of the nucleic acid include, for example, centrifugation, lysation of cells to release nucleic acid or boiling to break down cell walls.

The performance of the PCR amplification method is also well known in the art, and is described in, for example, US Patent Specification No. 4,683,195 and 4,683,202. Generally, PCR amplification involves three distinct stages: (i) denaturation; (ii) annealing and (iii) extension. In the method of this aspect of the present invention, we particularly prefer to use temperatures within the following ranges for these three stages:
(i) denaturing: between about 92 and 96 °C, preferably about 94°C;
(ii) annealing: between about 37 and 72 °C, preferably about 68°C; and
(iii) extension: between about 72 and 74 °C, preferably about 72°C.

We have also found that it is advantageous to subject the sample to an extended period of denaturation before starting the first annealing-extension cycle, and also to subject the sample to an extended period of extension at the end of the last cycle. Although the exact period of these extended denaturing and extension periods may depend on the sample and conditions used, we have found that a period of between about 2 and 30 minutes, preferably about 10 mintues for each such extended period, is sufficient.

In the method of this aspect of the present invention, the PCR amplification technique can be performed for as many cycles as is desired. As indicated above, a complete cycle includes each of the denaturing, annealing and extension periods. The number of cycles, or the overall length of the amplication period, is not essential to the present invention. We have found, however, that between 20 and 40 cycles are generally sufficient for the production of sufficient amplification product to determine the presence or absence of the desired nucleic acid in the sample. More particularly, we prefer to use 30 cycles of amplification.

The amplification products resulting from the PCR amplification may be visualised using any standard procedure for the visualisation of nucleic acid fragments, for example, running the fragments on an agarose gel, followed by appropriate staining of the fragments. A particularly suitable method for such visualisation involves running the amplification products on, for example, a 2% agarose gel, followed by staining of the fragments with ethidium bromide. Such techniques are well known in the art.

As indicated above, the presence of a positive control vector in the PCR amplification mixture prevents the occurrence of false negative results, that is it prevents a negative result from being given even in the presence of the target nucleic acid fragment. Three possibilities arise when the amplification products of a polymerase chain reaction mediated detection of a nucleic acid sequence are viewed:
1. Only one band is present, corresponding to the amplification product of the positive control vector alone. In this case, it is clear that the target nucleic acid fragment is not present in the sample tested.
2. Two bands are present, one corresponding to the amplification product of the positive control vector, and the other corresponding to the amplification product of the target nucleic acid sequence. In this case, it is clear that the target nucleic acid sequence has been successfully detected, and the PCR amplification has not been inhibited.
3. No bands are present corresponding to either of the amplification product of the positive control vector or to the amplification product of the target nucleic acid sequence. In this case, it must be assumed that the PCR amplification was inhibited in some manner and a conclusion cannot be drawn as to the presence or absence of the target nucleic acid sequence in the sample.

The present invention also provides the use of a positive control vector in the PCR mediated detection of a nucleic acid sequence. It is anticipated that the use of such a vector will be of great benefit in the detection of micro-organisms, or the nucleic acid components of micro-organisms, directly from sample obtained from a patient.

The present invention further provides a kit to be used in the PCR mediated detection of a nucleic acid in a sample, particularly in a clinical sample, which kit comprises a positive control vector specific for the nucleic acid to be detected and DNA extraction and amplification reagents.

The present invention is further described with reference to the following Examples.

### EXAMPLE 1

Detection of *Mycobacterium tuberculosis* or *Mycobacterium bovis*

### A. Preparation of a Positive Control Plasmid pPG10

The plasmid pPG10 was constructed starting from the publicly available plasmid pBLUESCRIPT SKII+™ [obtainable from Stratagene]. pBLUESCRIPT SKII+ contains a gene for ampicillin resistance, an *E*. *coli* origin of replication and the F1 origin of single stranded DNA replication.

Single stranded DNA of the plasmid pBLUESCRIPT SKII+ in which some of the thymidine residues in the nucleic acid were substituted by uracil, was obtained by transformation of the *E. coli* strain CJ236 [obtainable from BRL] using the helper phage KO7 [obtainable from Biorad]. Single stranded DNA was precipitated using ethylene glycol, and then purified by agarose gel electrophoresis using the techniques described by Sambrook et al in Molecular Cloning: A Laboratory Manual.

Mutant strand DNA, having the sequences shown for oligonucleotides A and B, below, containing the sequence of the two primers IS41 and IS43 [Plikyatis et al Mol. Cel. Probes 5 (1991) 215], was prepared using the T7 DNA polymerase obtainable from Biorad, followed by transformation of the mutagenesis products into the *E. coli* strain DH5α. Primers IS41 and IS43 recognise the repetitive element in the genome of the bacteria *Mycobacterium tuberculosis* and *Mycobacterium bovis* known as IS6110. The *E. coli* strain DH5α can eliminate DNA strands containing uracil, thereby automatically selecting for the mutant strand. DNA from the transformants was obtained by alkaline lysis and the mutants were detected by digestion of the DNA using the restriction enzymes SspI and EcoRI.

The two oligonucleotide primers were inserted into the plasmid with a distance of 1250 base pairs between them.

Mutagenic oligonucleotide A has the sequence [Sequence ID No. 1]:

CTC TTC CTT TTT CAA TCC TGC GAG CGT AGG CGT CGG ATT GAA GCA TTT
ATC

The restriction site for the nuclease SspI is shown in bold and the sequence of the oligonucleotide primer IS41 is underlined.

Mutagenic oligonucleotide B has the sequence [Sequence ID No. 2]:

GTT CGG CTG CGG CGA ATT CTG GCG TGG ACG CGG CTG AGG CGG TAA TAC
GGT TA

In the sequence of oligonucleotide B, the restriction site for the nuclease EcoRI is shown in bold script, and the underlined sequence is that of the oligonucleotide primer IS43.

Site directed mutagenesis of oligonucleotide A into the plasmid pBLUESCRIPT SK+ introduces the sequence of the primer IS41 into the SspI site of the plasmid at position 2850 of the plasmid sequence. The restriction site is deleted during this process. In a similar fashion, site directed mutagenesis of oligonucleotide B introduces the sequence of primer IS43 into the AluI restriction site of the plasmid pBLUESCRIPT SKII+ at position 1095 of the plasmid, thereby deleting that restriction site and creating a new EcoRI site.

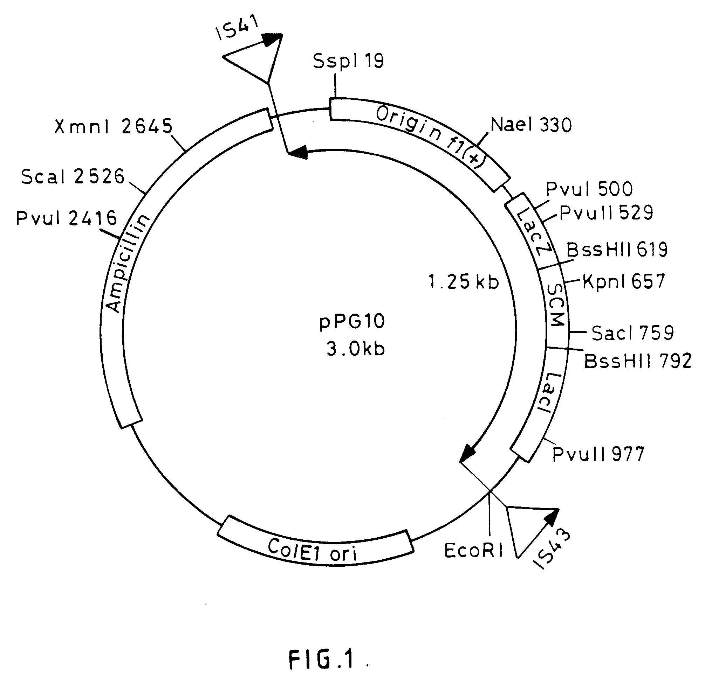

Plasmid pPG10 (Figure 1) is characterised by the lack of the SspI site and the AluI site of the parent plasmid, and the presence of the newly created EcoRI site at position 1095.

Plasmid pPG10 was then analysed using the polymerase chain reaction using the sequences of IS41 and IS43 as the primers, and the expected 1250 base pair amplification product was obtained.

### B. PCR Detection of Mycobacterium tuberculosis and Mycobacterium bovis

Sputum samples were treated with 30 ml of sodium hydroxide in order to lyse all micro-organisms other than Mycobacteria. The samples were then concentrated by centrifugation at 13 rpmp using standard procedures, and the pellet obtained was washed with saline, pelleted once again and then resuspended in water.

A 500 µl sample of the sputum suspension was pipetted into a 1.5 ml microfuge tube, and the sample was centrifuged 13 rpm. The pellet obtained was then washed with 1 ml of 10 mM tris(hydroxymethyl)aminomethane hydrochloric acid (Tris-HCl), pH 8.0, 1 mM ethylenediaminetetraacetic acid (EDTA) in tris-EDTA (TE) buffer and then centrifuged again. The pellet obtained was then resuspended in 75 µl of TE buffer containing 20 mg/ml of lysozyme, after which it was incubated at 37°C for 2 hours. At then end of this time, the Mycobacteria were lysed by treatment with 25 µl of 2 M NaOH and 5 µl of 20% sodium dodecyl sulfate (SDS) and boiling of the solution for 5 minutes. The samples were then neutralised with 50 µl of 1N aqueous hydrochloric acid and centrifuged again at 13 rpm.

PCR inhibitors were then removed from the sputum sample in the following manner. DNA was then extracted from the supernatant using the "Magic Miniprep"™ kit obtained from Promega, using standard techniques, with the exception that neutralisation was performed using 1 M aqueous hydrochloric acid instead of 2.55 M KOAc, pH 4.8, in order to avoid precipitation of genomic DNA. The column was then washed with 2 ml of a solution of 200 mM Tris-HCl, pH 7.5, 5 mM EDTA, 0.2 M sodium chloride and 50% ethanol, and the DNA was then eluted from the column in 50 µl water.

The polymerase chain reaction was then performed in a final volume of 50 µl of a solution comprising: 200 µM dATP, dCTP, dTTP and dGTP; 2.5 units of Taq Polymerase; 10 mM Tris-HCl (pH 8.3); 50 mM KCl; 1.5 mM MgCl₂; 0.5 µl of the primers and 10 pg of the control plasmid pPG10, prepared as described in step A above. 5 µl of the DNA purified from the sputum sample were then added to the reaction mixture. Before the start of the PCR reaction, the DNA was denatured for 10 minutes. Each PCR cycle consists of denaturing, annealing and extension and the temperatures and times for each cycle were as follows: denaturing for 2 minutes at 94°C; annealing for 2 minutes at 68°C and extension for 2 minutes at 72°C for 30 cycles. The time period for each step was increased by five seconds at the end of each cycle. At the end of the last cycle, the product was fully extended for a period of 10 minutes.

The PCR products were then analysed by running the samples on a 2% agarose gel and visualising by staining with ethidium bromide.

The positive control plasmid alone results in the production of a band having approximately 1250 base pairs, as described in step A, above. Successful amplification of the IS6110 target sequence results in the production of a band having approximately 317 base pairs. Therefore, when two bands are present, that is one band of 317 base pairs and one band equivalent to 1250 base pairs, then there has been successful amplification of the IS6110 target sequence indicating that the sample contained either *M. tuberculosis* or *M. bovis.* If, on the other hand, staining shows only one band equivalent to 1250 base pairs, this is an indication that there is no target IS6110 sequence in the sample, and therefore no Mycobacterium infection. If the result is no band at all, or at least no band equivalent to either of 317 or 1250 base pairs, then this is an indication that the PCR has been inhibited.

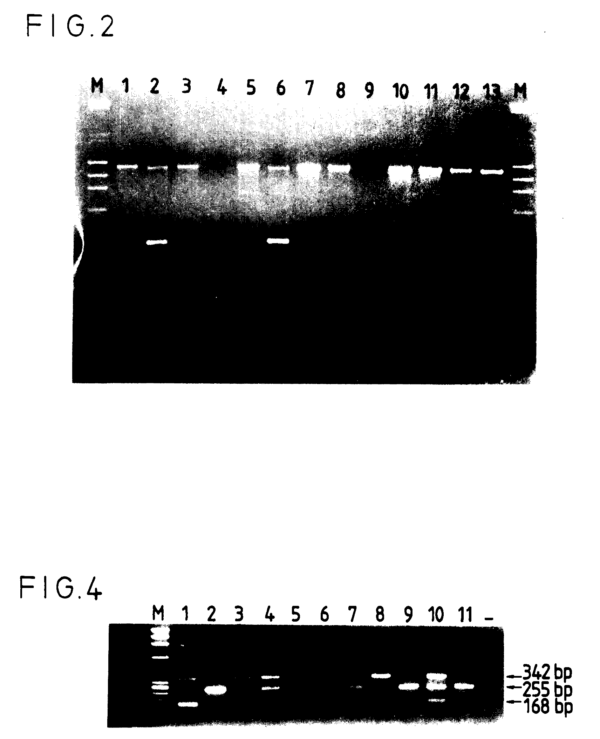

The results are shown in Figure 2. This figure shows the analysis of 13 clinical samples. A good correlation between microbiological and clinical data was obtained. The 1250 base pair band is present in all of the samples, except in columns 4 and 9, this therefore indicating that the PCR method did not work in those samples. The samples shown in columns 2 and 6 are the only to show a band of 317 base pairs, this indicating that these samples alone contained the Mycobacterium. The column M is the molecular weight marker.

### EXAMPLE 2

### Detection of micro-organisms causing periodontal disease

### A. Preparation of Primers

Primers were produced for the three micro-organisms known to be involved in periodontitis, namely *Actynomyces actynomycetemcomitans, Bacteroides intermedius* and *Bacteroides gingivalis.* DNA sequence alignment of the three micro-organisms was performed by using the information contained in the Gene Bank. The loci chosen for amplification is a highly variable region in the 16S ribosomal DNA, which contains a conserved sequence used to design the common primer U3, as discussed below. The specific oligonucleotides for each of the three micro-organisms, *Actynomyces actynomycetemcomitans, Bacteroides intermedius* and *Bacteroides gingivalis* were designed from a variable region within this loci, to amplify fragments of different sizes [Bi: 168 base pairs; Aa: 255 base pairs; and Bg: 342 base pairs]. The fact that each primer was specific for its micro-organism was proven using sequence alignment, using a DNA analysis program(Macaw®). This specificity was empirically demonstrated using DNA from other bacteria in the PCR amplification. In this case, no amplified product was obtained.

### B. Preparation of Plasmid pPGU3

Following a procedure similar to that described in Step A of Example 1, above, but using the mutagenic oligonucleotide MUTU3, having the sequence shown below [Sequence ID No. 3] to insert the sequence of the oligonucleotide primer U3 into the parent plasmid pBLUESCRIPT SKII+, the mutant plasmid pPGU3 was obtained.

Mutagenic oligonucleotide MUTU3:

GGC GAG CGG TAT CAG CTC GAG CGT GCC AGC AGC CGC GGT AAT ACG
CTC ACT CAA AGG CGG

Site directed mutagenesis of the sequence of U3 into the AluI site at position 1095 of pBLUESCRIPT SKII+ causes deletion of that site and creation of a new XhoI site (shown in bold). The sequence of the primer U3 is underlined. The plasmid is characterised by the presence of a new XhoI site and the lack of the AluI site.

The plasmid created by the site directed mutagenesis was called pPGMUTU3. The XhoI fragment of this plasmid was then sub-cloned using standard techniques, blunt ended using Klenow fragment DNA polymerase and then cut using the restriction enzyme EcoRI. This cut fragment was then inserted into pPGMUTU3 cut with the restriction enzymes SmaI and EcoRI.

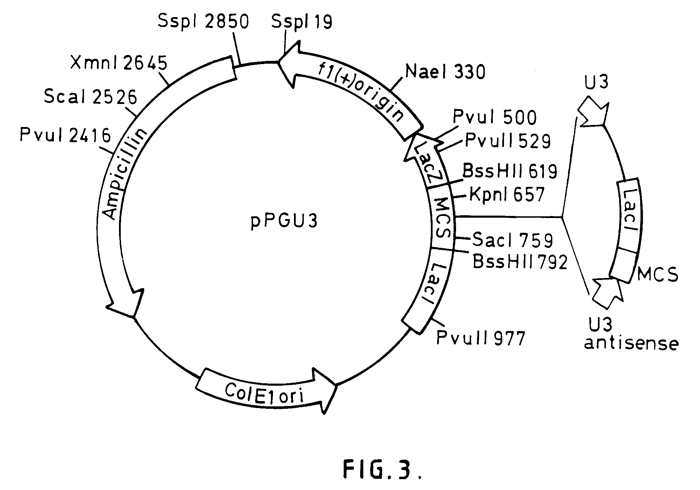

The resulting plasmid, pPGU3, the structure of which is shown in Figure 3, hereinafter, was then subjected to PCR amplification, resulting in the production of an amplification product of approximately 850 base pairs in length.

### C. Detection of bacteria causing periodontal disease in a sample

A sample was collected by the gentle insertion of a sterile, absorbent paper point (Johnson &Johnson, No. 2716) into the apical extent of the periodontal pocket or sulcus. After 10 seconds, the paper point was removed and then placed in a 1.5 ml Eppendorf tube containing 500 µl of saline solution. Micro-organisms in the sample of plaque were then eluted from the paper point by vortexing the tube for 30 seconds. After removal of the paper point, 20 µl of the sample were placed in a 700 µl tube and the solution was boiled for 5 minutes in order to disrupt the cells.

The primer U3, having the sequence [Sequence ID No. 4] shown underlined in Sequence ID No. 3, above is common for the three micro-organisms *Actynomyces actynomycetemcomitans, Bacteroides intermedius* and *Bacteroides gingivalis.* In order to distinguish between these three micro-organisms, the primers used in the PCR reaction were, individually, each specific for one of the micro-organisms. Thus, the primer AA2 [Sequence ID No. 5], having the sequence: CGT GCC AGC AGC CGC GGT AAT ACG
is specific for A. *actynomycetemcomitans* and results in the production of an amplification product in the PCR of 255 base pairs.

The primer BI7 [Sequence ID No. 6], having the sequence: CAT GAA TTC CGC CTG CGC TGC GTG TAC TCG GG
is specific for the micro-organism B. *intermedius* and results in the production of an amplification product of 168 base pairs in PCR.

The primer BG8 [Sequence ID No. 7], having the sequence: TTG GCC GCT TAC TGT ATA TCG CAA ACA GCG AG
is specific for the micro-organism *B. gingivalis* and results in the production of a PCR amplification product of 342 base pairs.

The solution used for the PCR had a final volume of 50 µl and consisted of: 200 µM dATP, dCTP, dGTP and dTTP; 2.5 units of Taq Polymerase; 10 mM Tris-HCl (pH 8.3); 50 mM KCl; 1.5 mM MgCl₂; 0.5 µM of a mixture of three primers, BG8, BI7 and AA2; 1.5 µM of the U3 primer, 1pg of the positive control plasmid pPGU3 and 5 µl of the boiled plaque sample. The mixture was then subjected to 30 cycles of the PCR, preceded by denaturing for 10 minutes. The cycles were as follows: denaturing for 2 minutes at 94°C; annealing for 2 minutes at 68°C and extension for 2 minutes at 72°C. The time period for each step was increased by five seconds at the end of each cycle. At the end of the last cycle, the product was fully extended for a period of 10 minutes.

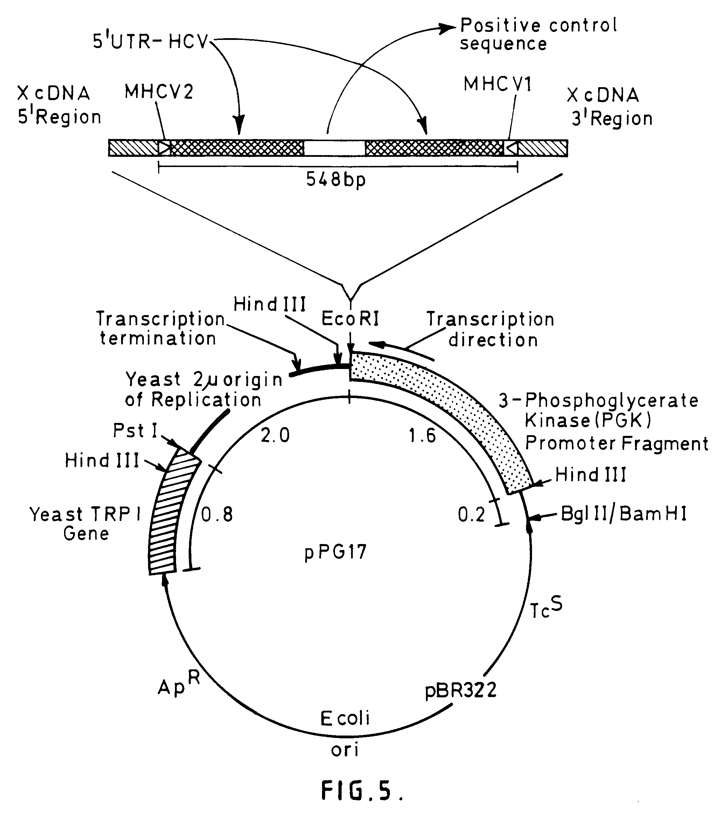

The PCR products were then analysed by running the samples on a 3% agarose gel and visualising by staining with ethidium bromide. The results are shown in Figure 4, hereinafter.

In clinical samples containing the three micro-organisms, the PCR amplification product will show bands at 168, 255, 342 and 850 base pairs. If only certain of the bacteria are present in the sample, then the bands corresponding to the amplified fragment specific for that bacteria will appear after staining. If none of the three micro-organisms in the sample, then only the band of 850 base pairs will appear. If the PCR reaction is inhibited, then there will be no bands at any of 168, 255, 342 or 850 base pairs.

In Figure 4, the results from amplification of DNA extracts from 11 clinical samples are shown. The column M is the molecular weight marker (φX174HaeIII marker), columns 1 to 11 are the samples containing one, two or three of the micro-organisms, and the column marked with a dash (-) is the negative control.

### EXAMPLE 3

### Detection of Hepatitis C in Blood Samples

### A. Construction of a PCR Positive Control Virus

The Hepatitis C virus 5' untranslated region was obtained by reverse transcriptase polymerase chain reaction amplification of a Hepatitis C containing sample using the primers MHCV1 and MHCV2, having the following sequences [Sequence ID No. 8 and 9]:

MHCV1: 5'-CCCGGGCGACACTCCACCATAGAT-3' (sense)

MHCV2: 5'-GGGCCCATGGTGCACGGTCTACGAG 3'(antisense -
reverse transcription primer)

In these sequences, the SmaI restriction site is shown in bold, and the sequence of the 5' untranslated region is underlined.

The reverse transcriptase PCR amplification product was then digested with SmaI, producing blunt ends, and cloned into the XmnI site of pRE89. pRE89 is a cDNA clone of X virus, a defective yeast double stranded RNA virus whose expression is driven by the yeast PGK1 promoter. The ligation product was transformed into the *E. coli* strain DH5α. The recombinants were selected by the PCR, using the sequence of the 5' untranslated region ("outer primers") as primers for the PCR.

The plasmid containing the 5' untranslated region of the Hepatitis C virus was called pPG15, and the construction was confirmed by restriction analysis and sequencing.

Next, an internal deletion spanning nucleotides -127 to -170 of the 5' untranslated region of the Hepatitis C virus conserved region was made in order to allow distinction to be made between the amplification products of the Hepatitis C virus and the positive control when using a reporter probe whose sequence is complementary to the deleted fragment. This step also allowed the introduction of a BamHI restriction site into the DNA in order to facilitate cloning. The internal deletion was created by oligonucleotide mutagenesis using the T7 DNA polymerase (Biorad) and the mutagenic primer MHCV3, having the sequence [Sequence ID No. 10]:

MHCV3: 5'-TGCAGCCTCCAGGATTCCAATTGCCAGGACGAC-3'

In the above sequence, the sequence of the newly introduced BamHI site is shown in bold and the sequence of the 5'-untranslated region is underlined.

The mutants were then screened using the PCR using the outer primers as primers for the reaction. The correct construction of this plasmid, called pPG16, was confirmed by sequencing.

Plasmid pPG16 was then used as a host for a 150 base pair fragment containing the sequence of the oligonucleotide positive control probe, this sequence being inserted into the BamHI site of pPG16 using standard techniques. The DNA fragment was obtained by the PCR from the plasmid pBLUESCRIPT SKII+ using the following nucleotide primers [Sequence ID No. 11 & 12]:

PBS11: 5'-CCTAGGTTCTATGGATAACCGTATTA-3'
PBS12: 5'-GGATCCGCATTAATGAATCGGCCAAC-5'

The BamHI site is shown in bold script.

The amplification product obtained by subjecting the pBLUESCRIPT SKII+ plasmid to the PCR was then restricted with the enzyme BamHI, and the resulting linear DNA was then inserted into the plasmid pPG16, which had previously been cut with BamHI. The resulting plasmid was then used to transform the *E*. *coli* strain DH5α, and the ligation mixture and transformants were screened by the PCR, using the Hepatitis C virus outer primers in the reaction. Finally, the construct was confirmed by sequence analysis.

The new recombinant plasmid, named pPG17, whose structure is shown in Figure 5, hereinafter, contained the positive control probe sequence inserted into the Hepatitis C virus 5' untranslated region, which is also inserted into the X virus cDNA clone, and its transcription was under the control of the yeast PGK1 promoter.

A single and double stranded RNA mutant viral genome was then obtained in the following manner. The yeast strain Y59 [obtainable from the American Type Culture Collection, ATCC] (alpha, trp1, ski2, L-A-HN, Mo) was transformed with the plasmid pPG17, prepared as described above, which encodes the recombinant X virus including the positive control sequences, and the transformants were selected by plating onto H-trp plates. The yeast strain Y59 contains the L-A helper virus which is know to supply the X major coat protein and the RNA dependent RNA polymerase. This yeast therefore allows the replication and encapsulation of the pPG17 transcripts containing the viral sequence. Transcription of pPG17 is driven by the PGK1 promoter, using the yeast RNA polymerase.

The selected transformants were then grown in a synthetic medium purchased from BRL without tryptophan for 2 days and at a temperature of 30°C. Viral particles were obtained by caesium chloride centrifugation, following the procedure described by Fujimurara & Wickner in J. Biol. Chem. 263 (1988) 454. The replicative forms of the mutant virus were separated by electrophoresis using standard techniques. Amplification of pPG17 using the PCR results in the production of a band equivalent to a fragment of 342 base pairs.

### B. Detection of Hepatitis C virus in a blood sample

RNA was isolated from blood samples following the conditions and techniques described by Cristiano et al in Hepatology 14 (1991) 51. Reverse transcriptase PCR (RT-PCR) was then performed using varying amounts of the positive control RNA prepared as described in Step A, above, that is amounts of between 10 and 10,000 molecules. The procedure for RT-PCR is as described in Cristiano et al, supra.

The amplification products were analysed in a 2% agarose gel stained with ethidium bromide. The Hepatitis C virus, if present in the sample, results in the production of a band equivalent to a fragment having 259 base pairs, whilst the amplification product of the positive control virus is, as stated above, equivalent to 342 base pairs. The two bands resulting when the Hepatitis C virus was present in the sample were further characterised by hybridization techniques, using the positive control virus and the 5' untranslated region of the Hepatitis C virus as oligonucleotide probes.

### EXAMPLE 4

### Detection and Quantitation of Interleukin-2 mRNA Levels in Human Cells

### A. Construction of a PCR Positive Control Recombinant Virus

A procedure similar to that described in Step A of Example 3, above was followed with the exception that the sequence introduced into the X cDNA clone consisted of two primer sequences, separated by approximately 500 base pairs.

Mutagenic oligonucleotide MIL2, having the following sequence [Sequence ID No. 13], in which the BamHI site is shown in bold script and the sequence of the IL2 primer is shown underlined, was used to introduce the Interleukin-2 (IL-2) sense and IL-2 anti-sense complementary sequence into the X cDNA clone PRE8.

MIL2: 5'-CATGTACAGCATGCAGCTCGCATCCGGATTCCATGATGAG
CCAGCAACTGTGGTGG-3'

This primer was converted into its double stranded form by use of the PCR, using oligonucleotides IL2S and IL2AS as the primers, and it was then ligated into pRE89 cut with the enzyme XmnI to form blunt ends. The resulting ligation mixture was then used to transform the E. *coli* strain DH5α, and the transformants were screened using restriction analysis. The construct was then further characterised by sequence analysis. The resulting plasmid was called pPG21.

Following the procedure outlined in Step A of Example 3, above, but using the mutagenic primer PBS15 [Sequence ID No. 14] instead of PBS12, a 500 base pair DNA fragment containing the positive control sequences was then inserted into the BamHI site of pPG21, prepared as described above.

PBS15: 5'-GGATCCACGACTCACTATAGGGCGAA-3'

The BamHI site is shown in bold script.

The mutant X cDNA clone thus resulting and containing the IL-2 primer sequences separated by 500 base pairs was called pPG22. Amplification of this plasmid using PCR and the IL-2 primer indicated that this positive control results in the production of a band of approximately 554 base pairs.

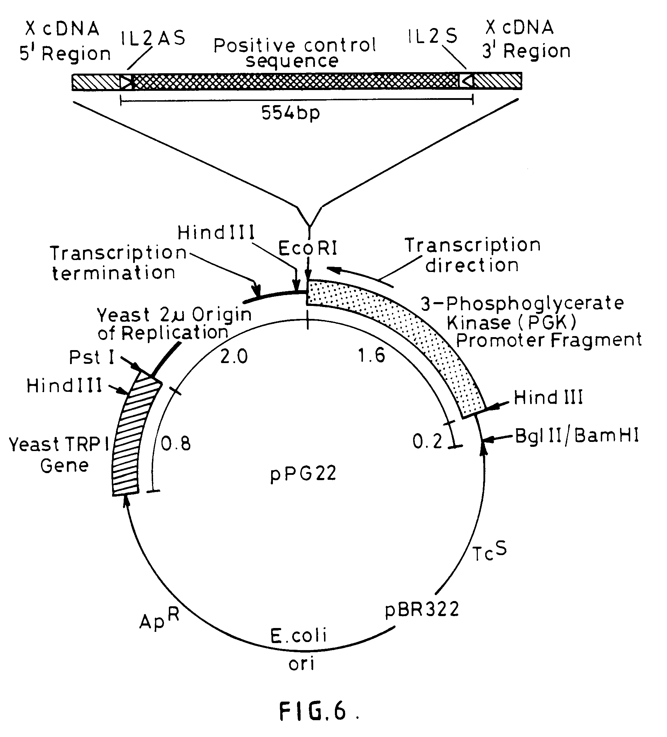

The plasmid pPG22, having the structure indicated in Figure 6, hereinafter and prepared as described above, was used to transform yeast cells in order to obtain the mutant virus genome containing the RNA sequences of the IL-2 positive control, using procedures similar to those described in Step A of Example 3, above.

RNA was isolated from human cells following procedures outlined by McKnight in Eur. J. Immunol 21 (1991) 1187, and the conditions for the RT-PCR used were also as described in that article. Between 10 and 10,000 molecules of the control RNA were added to the RNA samples for performance of the PCR. After visualisation of the bands obtained using 2% agarose gel stained with ethidium bromide. i.e. a band of 410 base pairs and a band of 554 base pairs, it was apparent that the presence of the positive control virus in the reaction ensured that a clean result was obtained.

### EXAMPLE 5

### Detection of M tuberculosis 65 KDa antigen mRNA

### A. Production of a Positive Control Recombinant Virus

The following two primers, described by Hance in Mol. Microbiol. 3 (1989) 843, were used in the construction of the positive control virus:

TB-1 [Sequence ID No. 15]: 5'-GAGATCGAGCTGGAGGATCC-3'
TB-2 [Sequence ID No. 16]: 5'-AGCTGCAGCCCAAAGGTGTT-3'

TB-1 is derived from bases 397 to 416 of the nucleic acid encoding the 65KDa antigen, and is the sense primer, whereas TB-2 is derived from bases 554 to 535 and is the antisense primer.

The positive control virus was constructed in a similar manner to that described in Step A of Example 4 above. The TB-1 and TB-2 sequences, separated by a BamHI restriction site, were inserted into the X cDNA clone pRE89 using the mutagenic oligonucleotide MTB65KD [Sequence ID No. 17]: 
MTB65KD: 5'-GAGATCGAGCTGGAGGATCCGGATTCCAACACCTTT
GGGCTGCAGCT-3'
in which the BamHI site is shown in bold script.

This primer was converted into its double stranded form by the PCR, using the oligonucleotides TB-1 and TB-2 as primers, and the product was then ligated using blunt end ligation into the XmnI site of pRE89.

The resulting plasmid was used to transform the DH5α strain of *E. coli* and the transformants were screened using restriction analysis. The construct was then further characterised using sequence analysis. The resulting plasmid was called pPG25.

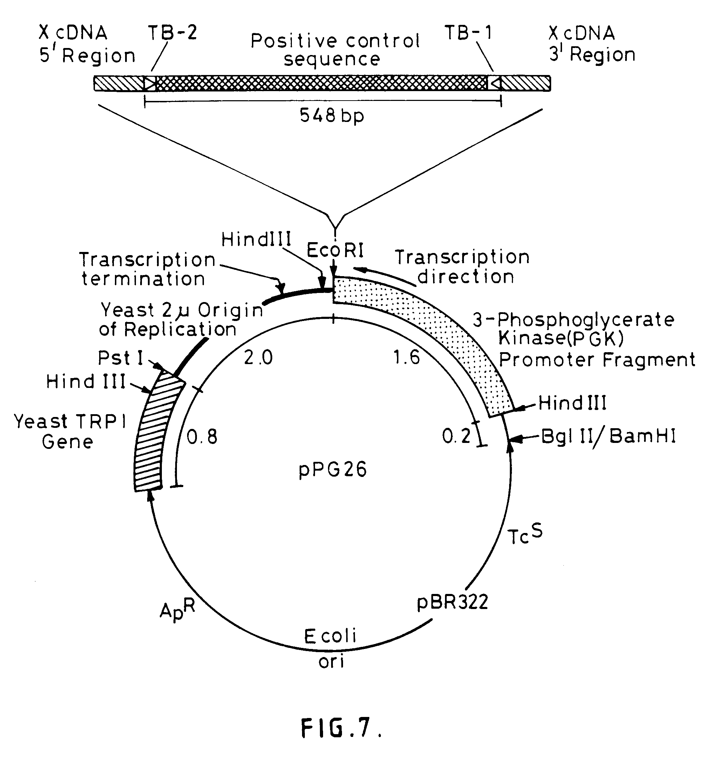

Following the procedure outlined in Step A of Example 4, above, the 500 base pair positive control sequence was obtained and inserted into plasmid pPG25. The resulting plasmid, pPG26, having the structure indicated in Figure 7, hereinafter, contained the sequences of the primers TB-1 and TB-2 separated by a 500 base pair sequence containing the positive control probe inserted into the X cDNA sequence. PCR amplification of this plasmid with the TB-1 and TB-2 sequences as primers, results in the production of a band of 548 base pairs.

Following the procedure outlined in Step A of Example 3, above, the mutant virus genome containing the RNA sequences of the M *tuberculosis* 65KDa antigen positive control was obtained by transformation of yeast cells with the plasmid pPG26.

### B. PCR Detection of the 65KDa Antigen of M tuberculosis in a sample

Mycobacterial cells were prepared for PCR by sonication in the presence of glass beads and phenol-chloroform. After extraction three times with the phenol-chloroform solution, the RNA was precipitated and re-dissolved in RNAse-free water. Varying amounts of the positive control RNA, that is between 10 and 10,000 molecules, were added to the PCR mixture, and RT-PCR was performed using standard techniques.

The amplification products were visualised by running through a 2% agarose gel and staining with ethidium bromide. The presence of bands of approximately 383 and 548 base pairs (that is corresponding to the 65KDa antigen and the positive control sequence respectively), indicates that the PCR has been clean and that false negative results have been avoided. The bands are then further characterised by hybridization using the positive control and TB-1 and TB-2 sequences as probes.

## Claims

1. A vector for use as a positive control in the detection of a particular nucleic acid sequence in a sample by the polymerase chain reaction amplification method, wherein the vector includes at least one nucleotide sequence comprising a sequence substantially identical to the sequence of a primer used in the polymerase chain reaction amplification method.

2. The vector of claim 1 in which said particular nucleic acid sequence is derived from the group consisting of: a micro-organism, the total nucleic acid of a cancerous cell, the genome of a single stranded or double stranded virus or a double stranded RNA virus transcript and particularly from a micro-organism.

3. The vector of claim 1 including from two to ten nucleotide sequences substantially identical to the sequence of a primer used in the polymerase chain reaction amplification method, particularly including two nucleotide sequences substantially identical to the sequence of a primer used in the polymerase chain reaction amplification method.

4. The vector of claim 1 in which said at least one nucleotide sequence is inserted into both the sense and anti-sense strands of said vector.

5. The vector of claim 1 in which there is a distance of less than 1.5 Kb between said at least one nucleotide sequence in the sense strand of the vector and said at least one nucleotide sequence in the anti-sense strand of the vector.

6. The vector of claim 1 which is selected from the group consisting of pPG10, pPGU3, pPG17, pPG22 and pPG26.

7. A method for the detection of a particular nucleic acid in a sample, which method involves performance of the polymerase chain reaction on a sample suspected of containing said particular nucleic acid sequence, in the presence of a positive control vector.

8. The method of claim 7 in which the sample is subjected to an extended period of denaturing before starting the first annealing-extension cycle, and is also subjected to an extended period of extension at the end of the last cycle.

9. The method of claim 7 in which the positive control vector is as defined in any one of claims 1 to 6.

10. A kit to be used in the polymerase chain reaction mediated detection of a nucleic acid in a sample, which kit comprises a positive control vector specific for said nucleic acid to be detected, as well as DNA extraction and amplification reagents.

11. A kit according to claim 10 wherein the vector is as defined in any one of claims 1 to 6.

12. A primer for use in the polymerase chain reaction mediated detection of the pathogens causing periodontitis.

13. The primer of claim 12 which enables detection of a microorganism selected from the group consisting of *Actynomyces actynomycetemcomitans, Bacteroides intermedius* and *Bacteroides gingivalis,* particularly a primer selected from:


SEQUENCE LISTING

(1) GENERAL INFORMATION:

(i) APPLICANT:
(A) NAME: PHARMA GEN S.A.
(B) STREET: CALLE DE CALERA, 3,
(C) CITY: MADRID
(E) COUNTRY: SPAIN
(F) POSTAL CODE (ZIP): 28760
(G) TELEPHONE: (91) 803 20 00
(H) TELEFAX: (91) 803 11 43

(ii) TITLE OF INVENTION: CONTROL OF PCR MEDIATED DETECTION OF
MICRO-ORGANISMS

(iii) NUMBER OF SEQUENCES: 17

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: GB 9217368.1
(B) FILING DATE: 14-AUG-1992

(vi) PRIOR APPLICATION DATA:
(A) APPLICATION NUMBER: GB 9303498.1
(B) FILING DATE: 22-FEB-1992

(vi) PRIOR APPLICATION DATA:
(A) APPLICATION NUMBER: GB 9309198.1
(B) FILING DATE: 05-MAY-1992

(vi) PRIOR APPLICATION DATA:
(A) APPLICATION NUMBER: GB 9309195.7
(B) FILING DATE: 05-MAY-1992

(2) INFORMATION FOR SEQ ID NO: 1:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: MYCOBACTERIUM TUBERCULOSIS

(xi) SEQUENCE DESCRIPTION: SEQ ID NO: 1:

CTCTTCCTTT TTCAATCCTG CGAGCGTAGG CGTCGGATTG AAGCATTTAT C 51

(2) INFORMATION FOR SEQ ID NO: 2:

(i) SEQUENCE CHARACTERISTICS:
(A) LENGTH: 53 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: Mycobacterium bovis

(xi) SEQUENCE DESCRIPTION: SEQ ID NO: 2:

GTTCGGCTGC GGCGAATTCT GGCGTGGACG CGGCTGAGGC GGTAATACGG TTA 53

(2) INFORMATION FOR SEQ ID NO: 3:

(i) SEQUENCE CHARACTERISTICS:
(A) LENGTH: 60 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: A. ACTYNOMYCETEMCOMITANS, B. INTERMEDIUS, B.
GINGIVALIS

(xi) SEQUENCE DESCRIPTION: SEQ ID NO: 3:

GGCGAGCGGT ATCAGCTCGA GCGTGCCAGC AGCCGCGGTA ATACGCTCAC TCAAAGGCGG 60

(2) INFORMATION FOR SEQ ID NO: 4:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: A. ACTYNOMYCETEMCOMITANS, B. INTERMEDIUS, B.
GINGIVALIS

(xi) SEQUENCE DESCRIPTION: SEQ ID NO: 4:

CGTGCCAGCA GCCGCGGTAA TACG 24

(2) INFORMATION FOR SEQ ID NO: 5:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: ACTYNOMYCES ACTYNOMYCETEMCOMITANS

(xi) SEQUENCE DESCRIPTION: SEQ ID NO: 5:

CGTGCCAGCA GCCGCGGTAA TACG 24

(2) INFORMATION FOR SEQ ID NO: 6:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: BACTEROIDES INTERMEDIUS

(xi) SEQUENCE DESCRIPTION: SEQ ID NO: 6:

CATGAATTCC GCCTGCGCTG CGTGTACTCG GG 32

(2) INFORMATION FOR SEQ ID NO: 7:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:

(A) ORGANISM: BACTEROIDES GINGIVALIS

(xi) SEQUENCE DESCRIPTION: SEQ ID NO: 7:

TTGGCCGCTT ACTGTATATC GCAAACAGCG AG 32

(2) INFORMATION FOR SEQ ID NO: 8:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: HEPATITIS C VIRUS

(xi) SEQUENCE DESCRIPTION: SEQ ID NO: 8:

CCCGGGCGAC ACTCCACCAT AGAT 24

(2) INFORMATION FOR SEQ ID NO: 9:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: YES

(vi) ORIGINAL SOURCE:
(A) ORGANISM: HEPATITIS C VIRUS

(xi) SEQUENCE DESCRIPTION: SEQ ID NO: 9:

GGGCCCATGG TGCACGGTCT ACGAG 25

(2) INFORMATION FOR SEQ ID NO: 10:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: HEPATITIS C VIRUS

(xi) SEQUENCE DESCRIPTION: SEQ ID NO: 10:

TGCAGCCTCC AGGATTCCAA TTGCCAGGAC GAC 33

(2) INFORMATION FOR SEQ ID NO: 11:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: HEPATITIS C VIRUS

(xi) SEQUENCE DESCRIPTION: SEQ ID NO: 11:

CCTAGGTTCT ATGGATAACC GTATTA 26

(2) INFORMATION FOR SEQ ID NO: 12:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: YES

(vi) ORIGINAL SOURCE:
(A) ORGANISM: HEPATITIS C VIRUS

(xi) SEQUENCE DESCRIPTION: SEQ ID NO: 12:

GGATCCGCAT TAATGAATCG GCCAAC 26

(2) INFORMATION FOR SEQ ID NO: 13:

(i) SEQUENCE CHARACTERISTICS:
(A) LENGTH: 56 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: INTERLEUKIN-2

(xi) SEQUENCE DESCRIPTION: SEQ ID NO: 13:

CATGTACAGC ATGCAGCTCG CATCCGGATT CCATGATGAG CCAGCAACTG TGGTGG 56

(2) INFORMATION FOR SEQ ID NO: 14:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: INTERLEUKIN-2

(xi) SEQUENCE DESCRIPTION: SEQ ID NO: 14:

GGATCCACGA CTCACTATAG GGCGAA 26

(2) INFORMATION FOR SEQ ID NO: 15:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: MYCOBACTERIUM TUBERCULOSIS 65KDA ANTIGEN

(x) PUBLICATION INFORMATION:
(A) AUTHORS: HANCE,
(C) JOURNAL: Mol. Microbiol.
(D) VOLUME: 3
(F) PAGES: 843-
(G) DATE: 1989
(K) RELEVANT RESIDUES IN SEQ ID NO: 15: FROM 1 TO 20

(xi) SEQUENCE DESCRIPTION: SEQ ID NO: 15:

GAGATCGAGC TGGAGGATCC 20

(2) INFORMATION FOR SEQ ID NO: 16:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: YES

(vi) ORIGINAL SOURCE:
(A) ORGANISM: MYCOBACTERIUM TUBERCULOSIS 65KDA ANTIGEN

(x) PUBLICATION INFORMATION:
(A) AUTHORS: HANCE,
(C) JOURNAL: Mol. Microbiol.
(D) VOLUME: 3
(F) PAGES: 843-
(G) DATE: 1989

(xi) SEQUENCE DESCRIPTION: SEQ ID NO: 16:

AGCTGCAGCC CAAAGGTGTT 20

(2) INFORMATION FOR SEQ ID NO: 17:

(i) SEQUENCE CHARACTERISTICS:
(A) LENGTH: 47 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(A) ORGANISM: MYCOBACTERIUM TUBERCULOSIS 65KDA ANTIGEN

(xi) SEQUENCE DESCRIPTION: SEQ ID NO: 17:

GAGATCGAGC TGGAGGATCC GGATTCCAAC ACCTTTGGGC TGCAGCT 47